# EUROPEAN PATENT APPLICATION

(11) **EP 1 935 888 A1**
(43) Date of publication of application: **25.06.2008**
(21) Application number: 06797992.2
(22) Date of filing: 14.09.2006
(51) Int. Cl.: C07D 261/20

(54) **METHOD FOR SULFONATING 1,2-BENZISOXAZOLE-3-ACETIC ACID**

(30) Priority: 16.09.2005 US 717207 P
(71) Applicant: Dainippon Sumitomo Pharma Co., Ltd., Osaka 541-8524 (JP)
(72) Inventor: ISHIKURA, Tsutomu, Suzuka-shi, Mie 5130818 (JP); HORIUCHI, Nobuhiko, Suita-shi, Osaka 5640053 (JP)
(74) Representative: Webster, Jeremy Mark
(86) International application number: PCT/JP2006/318280
(87) International publication number: WO 2007/032441

(57) **Abstract**

A method for efficiently preparing 1,2-benzisoxazole-3-methanesulfonic acid, which comprises reacting 1,2-benzisoxazole-3-acetic acid in toluene with chlorosulfonic acid, which may optionally be mixed with an inert solvent, in the presence of a specific Lewis base (ester or nitrile).

## Description

### TECHNICAL FIELD

The present invention relates to a method for selective sulfonation of 1,2-benzisoxazole-3-acetic acid in toluene with chlorosulfonic acid. Specifically, the present invention relates to a method for efficiently preparing 1,2-benzisoxazole-3-methanesulfonic acid, which comprises a step of reacting 1,2-benzisoxazole-3-acetic acid in toluene with chlorosulfonic acid in the presence of a specific Lewis base other than dioxane.

### BACKGROUND ART

1,2-Benzisoxazole-3-methanesulfonic acid prepared by sulfonation reaction of 1,2-benzisoxazole-3-acetic acid with chlorosulfonic acid is an intermediate for 1,2-benzisoxazole-3-methanesulfonamide being useful as an anti-epileptic agent.

Patent Document 1 and Non-patent Document 1 disclose a process for the preparation of 1,2-benzisoxazole-3-methanesulfonic acid, which comprises reacting chlorosulfonic acid and 1,2-benzisoxazole-3-acetic acid in the presence of dioxane in ethylene chloride (also known as 1,2-dichloroethane) or dichloromethane.

Patent Document 2 and Patent Document 3 disclose that when 1,2-benzisoxazole-3-acetic acid and chlorosulfonic acid are reacted in toluene, 1,2-benzisoxazole-3-methanesulfonic acid, the disulfonated benzisoxazole derivative as a by-product, and the starting compound (1,2-benzisoxazole-3-acetic acid) exist in the reaction mixture in the ratios of 63.5 %, 3 % and 32 %, respectively. However, these literatures never refer to the isolated yield of 1,2-benzisoxazole-3-methanesulfonic acid in this reaction, and the purity thereof.

Patent Document 4, Patent Document 1, Patent Document 5 and Non-patent Document 2 disclose a process for the preparation of 1,2-benzisoxazole-3-methanesulfonamide, which comprises reacting phosphorus oxychloride (also known as phosphoryl chloride) with sodium 1,2-benzisoxazole-3-methanesulfonate, which is isolated in the solid form, followed by reacting the resultant with ammonia gas in ethyl acetate.

Patent Document 6 discloses a method for the preparation of 1,2-benzisoxazole-3-methanesulfonamide, which comprises reacting sodium 1,2-benzisoxazole-3-methanesulfonate with SOCl₂/DMF in toluene, followed by reacting the resultant with ammonia gas.

Patent Document 7 discloses a process for the preparation of the 1,2-dichloroethane free crystals 1,2-benzoxazole-3-methanesulfonamide, which is prepared by using 1,2 -dichloroethane as a solvent.
- Patent Document 1:: JP-A-53-77057
- Patent Document 2:: US 6,841,683
- Patent Document 3:: US 2003/0144527 A1
- Patent Document 4:: US 4,172,896
- Patent Document 5:: JP-A-54-163823
- Patent Document 6:: US 6,936,720
- Patent Document 7:: US 6,900,333
- Non-Patent Document 1:: Yakugakuzasshi, 116, 533-547 (1996)
- Non-Patent Document 2:: J. Med. Chem., 22, 180 (1979)

### DISCLOSURE OF INVENTION

### PROBLEMS TO BE SOLVED BY INVENTION

The present inventors took note on toluene as a solvent to be used for preparing 1,2-benzisoxazole-3-methanesulfonamide, and have intensively studied on a one-pot method for preparing 1,2-benzisoxazole-3-methanesulfonamide from 1,2-benzisoxazole-3-acetic acid as the starting compound without isolating any intermediate. The merits of using toluene as a solvent are that the amount of comparatively high toxic chlorinated solvents such as 1,2-dichloroethane can be reduced, and that it is easy to remove water derived from an aqueous sodium hydroxide solution to be used in the manufacturing process by azeotropic distillation from the reaction system, and further that toluene is stable to sodium hydroxide, phosphorus oxychloride and ammonia, which are used as reagents.

However, it is known that an aromatic compound such as toluene easily reacts with chlorosulfonic acid to give a sulfonic acid derivative (e.g., S. Patai and Z. Rappoport, The chemistry of sulphonic acids, esters and their derivatives, page 354-355, 1991, John Willy & Sons). In fact, when chlorosulfonic acid was actually added into a mixture of toluene and 1,2-benzisoxazole-3-acetic acid, and the mixture is reacted, we have found that the reaction product from the reaction of toluene with chlorosulfonic acid, and 1,2-benzisoxazole-3-methanesulfonic acid existed in the ratios of 44 % and 25 %, respectively, and the starting compound (1,2-benzisoxazole-3-acetic acid) still remained in the ratio of 29 %, as is shown in Comparative Example 5. Thus, by a method of simply adding chlorosulfonic acid into a mixture of 1,2-benzisoxazole-3-acetic acid and toluene, and heating the mixture, it is not easy to efficiently obtain 1,2-benzisoxazole-3-methanesulfonic acid because the sulfonation reaction of toluene proceeds simultaneously as well. In addition, it has been known from the description of JP-A-53-77057, etc. to use dioxane as a Lewis base in the sulfonation reaction of 1,2-benzisoxazole-3-acetic acid, but a Lewis base having less toxicity and being able to be used instead of dioxane has not been known yet.

### MEANS FOR SOLVING THE PROBLEMS

The present inventors have intensively studied on a method for preparing 1,2-benzisoxazole-3-methanesulfonamide being industrially useful, especially a method for efficiently preparing 1,2-benzisoxazole-3-methanesulfonic acid by reacting chlorosulfonic acid and 1,2-benzisoxazole-3-acetic acid in toluene. As a result, the present inventors have found that the sulfonation reaction of 1,2-benzisoxazole-3-acetic acid with chlorosulfonic acid in toluene can proceed in a high yield by utilizing a specific Lewis base (e.g., ester or nitrile) other than dioxane. Further, they have found that by combining a method of diluting chlorosulfonic acid with an inert solvent (e.g., chlorinated solvents) and adding, the above sulfonation reaction can proceed in a higher yield, and they have finally completed the present invention.

An object of the present invention is to provide a method for preparing 1,2-benzisoxazole-3-methanesulfonic acid by reacting 1,2-benzisoxazole-3-acetic acid with chlorosulfonic acid in toluene, which comprises adding chlorosulfonic acid, which may optionally be mixed with an inert solvent, into a mixture of a Lewis base (ester or nitrile), toluene and 1,2-benzisoxazole-3-acetic acid, and heating the mixture. In addition, the present invention further provides a method for preparing an alkali metal salt of 1,2-benzisoxazole-3-methanesulfonic acid, which comprises adding chlorosulfonic acid, which may optionally be mixed with an inert solvent, into a mixture of a Lewis base (ester or nitrile), toluene and 1,2-benzisoxazole-3-acetic acid, heating the mixture, adding thereto an aqueous alkali metal hydroxide solution, and isolating the precipitated crystals from the reaction mixture. The present invention further provides a one-pot method for preparing 1,2-benzisoxazole-3-methanesulfonamide, which includes a method for preparing 1,2-benzisoxazole-3-methanesulfonic acid by adding chlorosulfonic acid, which may optionally be mixed with an inert solvent, into a mixture of a Lewis base (ester or nitrile), 1,2-benzisoxazole-3-acetic acid and toluene, and reacting the mixture. These objects and other objects and advantages will be apparent to a person skilled in the art from the following description.

The present invention provides the following methods.
[1] A method for preparing 1,2-benzisoxazole-3-methanesulfonic acid by reacting 1,2-benzisoxazole-3-acetic acid with chlorosulfonic acid in toluene, which comprises;
   adding chlorosulfonic acid, which may optionally be mixed with an inert solvent, into a mixture of a Lewis base selected from the group consisting of a C₂₋₅ saturated aliphatic monocarboxylic acid C₁₋₄ alkyl ester, a benzoic acid C₁₋₄ alkyl ester, a C₁₋₄ alkyl cyanide and benzonitrile, toluene and 1,2-benzisoxazole-3-acetic acid; and heating the mixture.
[2] The method according to the above [1], wherein the Lewis base is a C₂₋₅ saturated aliphatic monocarboxylic acid C₁₋₄ alkyl ester.
[3] The method according to the above [1], wherein the Lewis base is a C₂₋₅ saturated aliphatic monocarboxylic acid ethyl ester.
[4] The method according to the above [1], wherein the Lewis base is acetic acid ethyl ester or isobutyric acid ethyl ester.
[5] The method according to any one of the above [1]-[4], wherein the inert solvent is dichloromethane, chloroform, or 1,2-dichroloethane.
[6] A method for preparing an alkali metal salt of 1,2-benzisoxazole-3-methanesulfonic acid, which comprises;
   adding chlorosulfonic acid, which may optionally be mixed with an inert solvent, into a mixture of a Lewis base selected from the group consisting of a C₂₋₅ saturated aliphatic monocarboxylic acid C₁₋₄ alkyl ester, a benzoic acid C₁₋₄ alkyl ester, a C₁₋₄ alkyl cyanide and benzonitrile, toluene and 1,2-benzisoxazole-3-acetic acid;
   heating the mixture; and
   isolating the precipitated crystals from the reaction mixture after adding thereto an aqueous alkali metal hydroxide solution.
[7] The method according to the above [6], wherein the Lewis base is a C₂₋₅ saturated aliphatic monocarboxylic acid C₁₋₄ alkyl ester.
[8] The method according to the above [6], wherein the Lewis base is a C₂₋₅ saturated aliphatic monocarboxylic acid ethyl ester.
[9] The method according to the above [6], wherein the Lewis base is acetic acid ethyl ester or isobutyric acid ethyl ester.
[10] The method according to any one of the above [6]-[9], wherein the inert solvent is dichloromethane, chloroform, or 1,2-dichroloethane.
[11] A one-pot method for preparing 1,2-benzisoxazole-3-methane-sulfonamide, which comprises;
   (a) preparing a first mixture containing 1,2-benzisoxazole-3-methanesulfonic acid by a method according to any one of the above [1]-[5], and toluene;
   (b) adding an aqueous alkali metal hydroxide solution to the first mixture and removing water by azeotoropic distillation to give a second mixture;
   (c) adding phosphorous oxychloride to the second mixture and reacting the mixture to give a third mixture;
   (d) adding ammonia to the third mixture and reacting the mixture to give a fourth mixture containing 1,2-benzisoxazole-3-methane-sulfonamide; and
   (e) isolating 1,2-benzisoxazole-3-methanesulfonamide from the fourth mixture.

### BEST MODE FOR CARRYING OUT THE INVENNTION

1,2-Benzisoxazole-3-acetic acid is prepared according to the method disclosed in the literatures such as JP-A-53-77057, etc.
The "Lewis base" used in the present invention is an ester or a nitrile. More particularly, the Lewis base is a compound selected from the group consisting of a C₂₋₅ saturated aliphatic monocarboxylic acid C₁₋₄alkyl ester, a benzoic acid C₁₋₄ alkyl ester, a C₁₋₄ alkyl cyanide and benzonitrile, and among them, a C₂₋₅ saturated aliphatic monocarboxylic acid C₁₋₄ alkyl ester is preferable.

The "C₂₋₅ saturated aliphatic monocarboxylic acid C₁₋₄ alkyl ester" means a C₁₋₄ alkyl ester of a straight chain or branched chain saturated aliphatic monocarboxylic acid having 2-5 carbon atoms. For example, a C₂ saturated aliphatic monocarboxylic acid C₂ alkyl ester means ethyl acetate. The preferable C₂₋₅ saturated aliphatic monocarboxylic acid C₁₋₄ alkyl ester is ones having a straight chain C₁₋₄ alkyl moiety, and more preferable one is a C₂₋₅ saturated aliphatic monocarboxylic acid ethyl ester. Specific examples are ethyl acetate, ethyl propionate, ethyl butyrate, ethyl isobutyrate, ethyl valerate and ethyl pivalate, and among them, ethyl acetate and ethyl isobutyrate are more preferable.
The preferable examples of "benzoic acid C₁₋₄ alkyl ester" are ethyl benzoate and propyl benzoate.
The "C₁₋₄ alkyl cyanide" means a compound consisting of a straight chain or branched chain C₁₋₄ alkyl group and a cyano group, and specific samples are acetonitrile, ethyl cyanide, isopropyl cyanide and tert-butyl cyanide.

The "inert solvent" used in the present invention means a solvent that can mix with chlorosulfonic acid without reacting with it, and the preferable one is a chlorinated solvent, for example, dichloromethane, chloroform, and 1,2-dichloroethane.
The amount of toluene to be used in the sulfonation process of 1,2-benzisoxazole-3-acetic acid with chlorosulfonic acid is in the range of 5-20 ml to 1 g of 1,2-benzisoxazole-3-acetic acid, and preferably in the range of 8-10 ml.
The amount of chlorosulfonic acid to be used in the sulfonation process of 1,2-benzisoxazole-3-acetic acid with chlorosulfonic acid is in the range of 1.1-2.0 equivalents to 1 equivalent of 1,2-benzisoxazole-3-acetic acid, and preferably in the range of 1.2-1.5 equivalent. In the mixing procedure of chlorosulfonic acid with an inert solvent, the amount of the solvent to be used therein is preferably in the range of 2-3 ml to 1 g of chlorosulfonic acid.
The amount of the Lewis base to be used in the sulfonation process of 1,2-benzisoxazole-3-acetic acid with chlorosulfonic acid is in the range of 1.1-5.0 equivalents to 1 equivalent of 1,2-benzisoxazole-3-acetic acid, and preferably in the range of 2.0-3.5 equivalents.

Chlorosulfonic acid is preferably added into a mixture of a Lewis base, toluene and 1,2-benzisoxazole-3-acetic acid at a temperature from 0 to 30°C. The reaction temperature after the addition of chlorosulfonic acid is usually in the range of 60-90°C, and preferably in the range of 70-85°C.
The term of "alkali metal salt" includes potassium salt and sodium salt, and among them, sodium salt is preferable.
The "aqueous alkali metal hydroxide solution" includes an aqueous sodium hydroxide solution and an aqueous potassium hydroxide solution, etc., and among them, an aqueous sodium hydroxide solution is preferable.
In the step (c) of the above [11], it is preferable to add a tertiary amine such as triethylamine, and the reaction temperature is usually in the range of 75-85°C.
In the step (d) of the above [11], the "ammonia" is preferably ammonia gas, and the reaction temperature is preferably in the range of 30-60°C.

In the step (e) of the above [11], the isolation of 1,2-benzisoxazole-3-methanesulfonamide is carried out by a conventional method, such as by adding water to the reaction mixture, precipitating the crystals from a mixed solvent of toluene and water, and collecting the precipitated crystals by filtration.
The purity of 1,2-benzisoxazole-3-methanesulfonamide obtained by the method of the present invention can be improved more by recrystallization. The solvent for recrystallization is, for example, aqueous ethanol, aqueous isopropanol, etc., and among them, aqueous isopropanol is preferable, and the more preferable one is a 45-55 % aqueous isopropanol. In the present invention, a 55 % aqueous C₂₋₄ alcohol means a mixture of water of 55 parts by volume and a C₂₋₄ alcohol of 45 parts by volume. During the recrystallization process, if the azeotropic distillation disclosed in US 6,900,333 is performed, then there is obtained a crystal of 1,2-benzisoxazole-3-methanesulfonamide, which can meet the criteria of the residual solvents such as toluene, 1,2-dichloroethane, etc.

The present invention will be illustrated in more detail by the following Examples, but the present invention should not be construed to be limited thereto. The contents of the starting compound and the product therefrom, and the purity of the product are expressed by the results measured by high performance liquid chromatography.

### Example 1

To a mixture of 1,2-benzisoxazole-3-acetic acid (1.77 g), ethyl isobutyrate (3.48 g) and toluene (15 ml) was added dropwise chlorosulfonic acid (1.40 g) with stirring at room temperature, and the resulting mixture was heated with stirring at 80°C for 120 minutes. It was confirmed that 1,2-benzisoxazole-3-methanesulfonic acid, the starting compound and the by-product existed in the reaction mixture in the ratios of 70 %, 21 % and 8 %, respectively. Water was added to the reaction mixture, and the desired compound was extracted into the aqueous layer. The pH value of the aqueous layer was adjusted to pH 10 with a 25 % aqueous sodium hydroxide solution, and the mixture was concentrated under reduced pressure. To the residue was added water so that the total weight of the mixture became 7 g, and the mixture was cooled with ice. The precipitated crystals were collected by filtration and dried at room temperature to give sodium 1,2-benzisoxazole-3-methanesulfonate (1.23 g, purity: 99 %).

### Examples 2-9

1,2-Benzisoxazole-3-acetic acid (1.77 g) was reacted and treated in a similar manner to Example 1 to give the following results as indicated in Table 1.

**Table 1**

| Ex. | Toluene | Chlorosulfonic acid | Lewis base | Contents in the reaction mixture | | | Yield (Purity) |
|---|---|---|---|---|---|---|---|
| | | | | Desired compd. | Starting compd. | By-product¹⁾ | |
| 1 | 15 ml | 1.40 g | Ethyl isobutyrate 3.48 g | 70% | 21% | 8% | 1.23 g (99 %) |
| 2 | 15 ml | 1.40 g | Ethyl acetate 2.64 g | 67% | 26% | 6% | 1.48 g (90 %) |
| 3 | 15 ml | 1.75 g | Ethyl acetate 2.64 g | 64% | 23% | 12% | 1.75g (94 %) |
| 4 | 15 ml | 1.40 g | Ethyl propionate 3.06 g | 63% | 28% | 7% | 1.29 g (93 %) |
| 5 | 15 ml | 1.40 g | Methy pivalate 3.48 g | 60% | 25% | 8% | 1.21 g (93 %) |
| 6 | 15 ml | 1.40 g | Eethy pivalate 3.91 g | 62% | 29% | 9% | - |
| 7 | 15 ml | 1.40 g | Ethyl benzoate 4.51 g | 63% | 28% | 9% | 1.29 g (91 %) |
| 8 | 18 ml | 1.40 g | Benzonitrile 1.55 g | 53% | 23% | 8% | 1.25 g (91 %) |
| 9 | 18 ml | 1.40 g | tert-Butyl cyanide 1.25 g | 66% | 23% | 8% | 1.12 g (92 %) |

| | | | | | | | |
|---|---|---|---|---|---|---|---|
| 1) The reaction product of toluene with chlorosulfonic acid | | | | | | | |

### Comparative Examples 1-5

1,2-Benzisoxazole-3-acetic acid (1.77 g) was reacted and treated in a similar manner to Example 1 to give the following results as indicated in Table 2.

**Table 2**

| Comparative Ex. | Toluene | Chlorosulfonic acid | Lewis base | Contents in the reaction mixture | | | Yield (Purity) |
|---|---|---|---|---|---|---|---|
| | | | | Desired compd. | Starting compd. | By- product¹⁾ | |
| 1 | 18 ml | 1.40 g | Dioxane 1.32 g | 81 % | 12 % | 5 % | 1.59 g (92 %) |
| 2 | 15 ml | 1.40 g | Diethoxyethane 3.54 g | 51 % | 24 % | 5%,18%²⁾ | - |
| 3 | 18 ml | 1.40 g | Dimethylformamide 1.10 g | 3 % | 88 % | 9 % | - |
| 4 | 36 ml | 2.10 g | 1-Methylpyrrolidone 2.23 g | 4% | 80 % | 13 % | - |
| 5 | 18 ml | 2.33 g | None | 25% | 29% | 44% | - |

| | | | | | | | |
|---|---|---|---|---|---|---|---|
| 1) The reaction product of toluene with chlorosulfonic acid 2) By-products other than the reaction product of toluene with chlorosulfonic acid | | | | | | | |

### Example 10

To a mixture of 1,2-benzisoxazole-3-acetic acid (1.77 g), ethyl acetate (2.64 g) and toluene (15 ml) was added dropwise a solution of chlorosulfonic acid (1.75 g) in dichloromethane (5 ml) with stirring at room temperature, and the resulting mixture was heated with stirring at 70°C for 120 minutes. It was confirmed that 1,2-benzisoxazole-3-methanesulfonic acid, the starting compound and the by-product existed in the reaction mixture in the ratios of 84 %, 5 % and 7 %, respectively. Water was added to the reaction mixture, and the desired compound was extracted into the aqueous layer. The pH value of the aqueous layer was adjusted to pH 10 with a 25 % aqueous sodium hydroxide solution, and the mixture was concentrated under reduced pressure. To the residue was added water so that the total weight of the mixture became 7 g, and the mixture was cooled with ice. The precipitated crystals were collected by filtration and dried at room temperature to give sodium 1,2-benzisoxazole-3-methanesulfonate (2.38 g, purity: 96 %).

### Examples 11-13

1,2-Benzisoxazole-3-acetic acid (1.77 g) was reacted and treated in a similar manner to Example 10 to give the following results as indicated in Table 3.

**Table 3**

| Ex. | Toluene | Chlorosulfonic acid | Solvent | Lewis base | Contents in the reaction mixture | | | Yieid (Purity) |
|---|---|---|---|---|---|---|---|---|
| | | | | | Desired compd. | Starting compd. | By- product ¹⁾ | |
| 10 | 15 ml | 1.75 g | Dichloromethane 5 ml | Ethyl acetate 2.64 g | 84% | 5% | 7% | 2.38 g (96%) |
| 11 | 15 ml | 1.75 g | Chloroform 5 ml | Ethyl acetate 2.64 g | 86 % | 4 % | 6 % | 2.41 g (96%) |
| 12 | 15 ml | 1.75 g | 1,2-Dichloroethane 5 ml | Acetonitrile 1.23 g | 78% | 5% | 9% | - |
| 13 | 15 ml | 2.10 g | 1,2-Dichloroethane 5 ml | tert-Butyl cyanide 2.49 g | 74 % | 11 % | 12 % | - |

| | | | | | | | | |
|---|---|---|---|---|---|---|---|---|
| 1) The reaction product of toluene with chlorosulfonic acid | | | | | | | | |

### Example 14

1) To a mixture of 1,2-benzisoxazole-3-acetic acid (10.6 g), ethyl acetate (15.9 g) and toluene (90 ml) was added dropwise a solution of chlorosulfonic acid (10.5 g) in 1,2-dichloroethane (30 ml) with stirring over a period of 15 minutes at room temperature, and the mixture was stirred at room temperature for 20 minutes. After stirring at 70-80°C for 2 hours, ethyl acetate was removed by distillation, and the pH value of the residue was adjusted to pH 10 with a 25 % aqueous sodium hydroxide solution. To the mixture was added toluene (60 ml), and the mixture was concentrated under reduced pressure, and these procedures were repeated 3 times. Then, toluene (70 ml), triethylamine (1.3 g) and phosphorous oxychloride (9.2 g) were added to the residue, and the mixture was heated at 77-83°C with stirring for 6 hours. After cooling, to the reaction mixture was added toluene (50 ml), and ammonia gas was blown to the mixture until saturated, while the reaction temperature was kept at 30-60°C. The reaction mixture was concentrated, and water (100 ml) and toluene (15 ml) were added thereto. The mixture was stirred, and the precipitated crystals were collected by filtration, and washed with water to give the crude crystals of 1,2-benzisoxazole-3-methanesulfonamide. The purity of the crude crystals was 96.5 %.
2) The above crude crystals were recrystallized from a 50 % aqueous isopropanol (140 ml) to give the product (9.7 g, purity: 99.8 %). The crystals were recrystallized again from a 50 % aqueous isopropanol (60 ml), and dried at room temperature to give the crystals of 1,2-benzisoxazole-3-methanesulfonamide (6.7 g, purity: 100 %).

### INDUSTRIAL APPLICABILITY

Toluene is usually not suitable as a solvent for the sulfonation reaction because toluene easily reacts with chlorosulfonic acid. However, as mentioned above, it was found that the reaction of 1,2-benzisoxazole-3-acetic acid and chlorosulfonic acid can be promoted selectively by reacting in the presence of a specific Lewis base (ester or nitrile) other than dioxane. By this method, there is provided a one-pot method for efficiently preparing 1,2-benzisoxazole-3-methanesulfonamide by utilizing 1,2-benzisoxazole-3-acetic acid as a starting compound and toluene as a solvent.

## Claims

1. A method for preparing 1,2-benzisoxazole-3-methanesulfonic acid by reacting 1,2-benzisoxazole-3-acetic acid with chlorosulfonic acid in toluene, which comprises;
adding chlorosulfonic acid, which may optionally be mixed with an inert solvent, into a mixture of a Lewis base selected from the group consisting of a C₂₋₅ saturated aliphatic monocarboxylic acid C₁₋₄ alkyl ester, a benzoic acid C₁₋₄ alkyl ester, a C₁₋₄ alkyl cyanide and benzonitrile, toluene and 1,2-benzisoxazole-3-acetic acid; and
heating the mixture.

2. The method according to claim 1, wherein the Lewis base is a C₂₋₅ saturated aliphatic monocarboxylic acid C₁₋₄ alkyl ester.

3. The method according to claim 1, wherein the Lewis base is a C₂₋₅ saturated aliphatic monocarboxylic acid ethyl ester.

4. The method according to claim 1, wherein the Lewis base is acetic acid ethyl ester or isobutyric acid ethyl ester.

5. The method according to any one of claims 1-4, wherein the inert solvent is dichloromethane, chloroform, or 1,2-dichroloethane.

6. A method for preparing an alkali metal salt of 1,2-benzisoxazole-3-methanesulfonic acid, which comprises;
adding chlorosulfonic acid, which may optionally be mixed with an inert solvent, into a mixture of a Lewis base selected from the group consisting of a C₂₋₅ saturated aliphatic monocarboxylic acid C₁₋₄ alkyl ester, a benzoic acid C₁₋₄ alkyl ester, a C₁₋₄ alkyl cyanide and benzonitrile, toluene and 1,2-benzisoxazole-3-acetic acid;
heating the mixture; and
isolating the precipitated crystals from the reaction mixture after
adding thereto an aqueous alkali metal hydroxide solution.

7. The method according to claim 6, wherein the Lewis base is a C₂₋₅ saturated aliphatic monocarboxylic acid C₁₋₄ alkyl ester.

8. The method according to claim 6, wherein the Lewis base is a C₂₋₅ saturated aliphatic monocarboxylic acid ethyl ester.

9. The method according to claim 6, wherein the Lewis base is acetic acid ethyl ester or isobutyric acid ethyl ester.

10. The method according to any one of claims 6-9, wherein the inert solvent is dichloromethane, chloroform, or 1,2-dichroloethane.

11. A one-pot method for preparing 1,2-benzisoxazole-3-methanesulfonamide, which comprises;
(a) preparing a first mixture containing 1,2-benzisoxazole-3-methanesulfonic acid by a method according to any one of claim 1-5, and toluene;
(b) adding an aqueous alkali metal hydroxide solution to the first mixture and removing water by azeotoropic distillation to give a second mixture;
(c) adding phosphorous oxychloride to the second mixture and reacting the mixture to give a third mixture;
(d) adding ammonia to the third mixture and reacting the mixture to give a fourth mixture containing 1,2-benzisoxazole-3-methane-sulfonamide; and
(e) isolating 1,2-benzisoxazole-3-methanesulfonamide from the fourth mixture.
